Europäisches Patentamt

European Patent Office

Office européen des brevets

(11) Veröffentlichungsnummer: **0 233 510**
B1

# (12) EUROPÄISCHE PATENTSCHRIFT

(45) Veröffentlichungstag der Patentschrift:
11.04.90

(21) Anmeldenummer: 87101006.2

(22) Anmeldetag: 24.01.87

(51) Int. Cl.⁴: **C07C 211/10**, C07C 209/24,
C07C 209/26

(54) Verfahren zur Herstellung von Di-tert.-butyl-ethylendiamin.

(30) Priorität: 29.01.86 DE 3602527

(43) Veröffentlichungstag der Anmeldung:
26.08.87 Patentblatt 87/35

(45) Bekanntmachung des Hinweises auf die Patenterteilung:
11.04.90 Patentblatt 90/15

(84) Benannte Vertragsstaaten:
BE DE FR GB IT NL

(56) Entgegenhaltungen:
DE-A- 3 128 810
US-A- 2 809 995
US-A- 3 652 672
US-A- 4 160 785

PATENT ABSTRACTS OF JAPAN, Band 6,
Nr. 214 (C-131)[1092], 27. Oktober 1982; & JP - A
- 57 120 552
HOUBEN WEYL "Methoden der Organischen
Chemie", 4. Auflage, Band IV/1c, 1980, Seiten 228-237,
Georg Thieme Verlag, Stuttgart

(73) Patentinhaber: BASF Aktiengesellschaft,
Carl-Bosch-Strasse 38, D-6700 Ludwigshafen(DE)

(72) Erfinder: Müller, Herbert, Dr., Carostrasse 53,
D-6710 Frankenthal(DE)
Erfinder: Mesch, Walter, Dr., Richinesstrasse 11,
D-6700 Ludwigshafen(DE)

## Beschreibung

Die Erfindung betrifft ein Verfahren zur Herstellung von Di-tert.-butyl-ethylendiamin durch Umsetzung von tert.-Butylamin mit Glyoxal unter Bildung von Di-tert.-butyl-glyoxaldiimin und Wasser und anschließende Hydrierung des Diimins. Das Di-tert.-butyl-ethylendiamin ist ein wertvolles Zwischenprodukt, das beispielsweise bei der Herstellung von Polyphenylenoxidharzen als Katalysator brauchbar ist.

In der US-A-4 160 785 ist ein Verfahren der eingangs genannten Art beschrieben, bei dem in Gegenwart von Methanol gearbeitet wird. Bei dieser Arbeitsweise wird, ebenso wie in Tetrahedron 26, Seiten 2555 bis 2560 (1970) beschrieben, das Diimin aus tert.-Butylamin und wäßrigem Glyoxal hergestellt. Vor der Hydrierung muß das Diimin aus der Reaktionsmischung isoliert werden. Die Hydrierung nach der Arbeitsweise der US-A-4 160 785 erfolgt in Gegenwart von Methanol oder Ethanol. Entscheidend ist nach der Lehre der Druckschrift, daß ein Lösungsmittel für die Hydrierung gewählt wird, das eine hohe Wasserstoffaufnahme aufweist. Ausweislich der Tabelle I im Beispiel 3 der Druckschrift sind hierzu lediglich Methanol und Ethanol geeignet. Kohlenwasserstoffe, wie Cyclohexan und Benzol werden auf der Grundlage der Wasserstoffaufnahme als völlig ungeeignet qualifiziert. Nach dem Verfahren der US-A-4 160 785 erhält man Ausbeuten, die bei etwa 70 % liegen. Ausweislich der genannten Tabelle I erhält man bei Verwendung von Cyclohexan oder Benzol keine Hydrierausbeute.

Der Erfindung liegt die Aufgabe zugrunde, ein Verfahren der eingangs genannten Art zur Verfügung zu stellen, bei dem die Stufe der Umsetzung des tert.-Butylamins mit Glyoxal und die anschließende Hydrierstufe wesentlich vereinfacht und verbessert werden und höhere Gesamtausbeuten erzielbar sind.

Diese Aufgabe wird gelöst mit einem Verfahren der eingangs genannten Art, das dadurch gekennzeichnet ist, daß man

(a) die Umsetzung des tert.-Butylamins mit dem Glyoxal in Gegenwart einer Kohlenwasserstoffphase durchführt,

(b) den Kohlenwasserstoff mit dem Umsetzungsprodukt von Wasser abtrennt, und

(c) das Umsetzungsprodukt in der Kohlenwasserstoffphase katalytisch hydriert.

Nach einer bevorzugten Ausführungsform löst man das tert.-Butylamin im Kohlenwasserstoff und setzt das Glyoxal in wäßriger Lösung ein.

Als Kohlenwasserstoffe kommen insbesondere solche in Betracht, die unter den Bedingungen der Hydrierung des Diimins nicht hydrierbar sind. Bevorzugt sind Kohlenwasserstoffe, welche in einem Bereich von > 45°C bis etwa 160°C, insbesondere zwischen 60 und 130°C sieden.

Als Kohlenwasserstoffe kommen eine Vielzahl von Produkten in Betracht. Bevorzugt sind die preiswert industriell zugänglichen Kohlenwasserstoffe, beispielsweise cyclische Kohlenwasserstoffe, z.B. Cyclohexan, acyclische Kohlenwasserstoffe, beispielsweise Benzine, z.B. höhersiedende Benzinfraktionen mit Siedepunkten von beispielsweise 100 bis 120°C, andere industriell zugängliche Kohlenwasserstoffegemische, beispielsweise Produkte wie Skellysolve®. Besonders bevorzugt sind auch aromatische Kohlenwasserstoffe, beispielsweise Toluol. Besonders bevorzugt ist Xylol.

Erfindungsgemäß können sowohl einzelne Kohlenwasserstoffe oder Gemische von Kohlenwasserstoffen eingesetzt werden. Wesentlich ist nur, daß sich die eingesetzten Kohlenwasserstoffe gut von Di-tert.-butylethylendiamin trennen lassen. Das gilt insbesondere dann, wenn das Di-tert.-butylethylendiamin destillativ abgetrennt werden soll.

Die Eintopfreaktion mit Glyoxal, t-Butylamin, Kohlenwasserstoff wird besonders vorteilhaft so durchgeführt, daß man 2 bis 3 Molteile Amin in der halben bis doppelten Gewichtsmenge Kohlenwasserstoff löst und ein Molteil Glyoxal als wäßrige, etwa 40 bis 50 %ige Lösung zulaufen läßt. Die beiden Phasen werden durch einen Rührer gemischt. Die Umsetzung ist bei 50°C in 2 Stunden beendet.

Man trennt dann die Ölphase vom Wasser. Die klaren, homogenen Phasen trennen sich leicht voneinander. Es kann zweckmäßig sein, beispielsweise zur Vermeidung eines festen Hydrates von DTEB, das wenige, noch gelöste Wasser vor der Hydrierung aus der Ölphase herauszudestillieren. Es wird mit 5 bis 10 % des eingesetzten Kohlenwasserstoffs herausgeschleppt.

Die Hydrierung kann kontinuierlich oder diskontinuierlich erfolgen. Wenn die Hydrierung diskontinuierlich durchgeführt wird, so erweist es sich als vorteilhaft, in Gegenwart eines Suspensionskatalysators zu arbeiten. Bei kontinuierlicher Durchführung des Verfahrens ist die Verwendung eines Trägerkatalysators vorteilhaft.

Als Katalysator eignen sich Übergangsmetalle allgemein. Besonders geeignet sind Katalysatoren auf der Basis von Nickel, Platin und Palladium. Die Katalysatoren können übliche Promotoren und andere Zusätze enthalten.

Die erste Stufe der Reaktion wird zweckmäßig bei Temperaturen zwischen Raumtemperatur und 150°C durchgeführt. Die anschließende Hydrierung erfolgt bevorzugt bei Temperaturen zwischen 50 und 150°C, insbesondere bei Temperaturen von 60 bis 80°C.

Man arbeitet zweckmäßig bei Drücken von 10 bis 60 bar. In diesem Druckbereich ist die Aufnahme von Wasserstoff stürmisch. Es kann erforderlich sein, das System zu kühlen.

Das Hydrierprodukt wird in üblicher Weise aufgearbeitet, insbesondere durch fraktionierte Destillation. Durch die Aufarbeitung wird das Amin farblos. Man erhält Produkt mit Reingehalten von über 98 %. Die Ausbeute liegt hierbei bei etwa 85 % der Theorie.

Im Hinblick auf die Lehre der US-A-4 160 785 muß als überraschend gelten, daß das erfindungsgemäße Verfahren bei einfacher Durchführung zu reinem Produkt in hohen Ausbeuten führt. Die Angaben in der US-A-4 160 785, insbesondere Beispiel 3 und Tabelle I mußten beim Fachmann ein Vorurteil gegen den Einsatz von Kohlenwasserstoffen beim erfindungsgemäßen Verfahren entstehen lassen. Besonders überraschend ist beim erfindungsgemäßen Verfahren die stürmische Wasserstoffaufnahme in der Hydrierstufe, die häufig eine gesonderte Kühlung erforderlich macht. Das steht im besonders krassem Gegensatz zu den Ergebnissen in Tabelle I der US-A 4 160 785.

Man kann bisweilen die Ausbeute an Di-tert.-butylethylendiamin bis auf 90 % der Theorie dadurch steigern, daß man der Lösung des Diimins im Kohlenwasserstoff noch etwas tert.-Butylamin, beispielsweise etwa 5 Gew.-%, zusetzt.

Die Erfindung wird durch die nachstehenden Beispiele weiter erläutert.

Beispiel 1

400 g einer Benzinfraktion, Sp. 122 bis 124°C, mit dem Hauptbestandteil n-Octan werden mit 483 g t-Butylamin (6,6 g Mol) versetzt. Es entsteht eine klare Lösung.

Bei 45 bis 50°C werden dazu 435 g Glyoxal, wäßrig, 40 %ig (3,0 g Mol) zugetropft unter Rühren. Es wird eine Stunde nachgerührt.

Die spezifisch schwerere Wasserphase wird dann in einem Scheidetrichter von der Ölphase (922 g) getrennt.

Diese wird zur völligen Entwässerung andestilliert. 18 g Wasser und 21 g t-Butylamin werden neben 60 g Benzin als zweiphasiges Gemisch erhalten.

Kontakt für die Hydrierung der Ölphase ist Platin auf Kohle (5 %ig), 3 g, bei 80°C und 60 bar).

Durch Destillation erhält man 413 g Di-t-Butylethylendiamin (80 % d.Th. vom Sp. 120°C, 130 mbar). Es ist flüssig und nach GC 98,5 %ig.

Beispiel 2

435 g o-Xylol und 483 g t-Butylamin werden bei 45 bis 50°C mit 435 g Glyoxal, wäßrig, 40 %ig unter Rühren versetzt. Nach 1 Stunde wird die Ölphase abgetrennt und die Wasserphase nochmals mit 50 g Xylol ausgeschüttelt.

Aus den vereinigten Kohlenwasserstoffphasen werden bei 130 mbar bis zu einer Sumpftemperatur von 95°C 20 g Wasser ausgekreist.

Die Hydrierung geschieht mit 3 g Platin auf Kohle (5 %ig) bei 70°C und 60 bar Wasserstoffdruck. Die berechnete Menge Wasserstoff ist in zwei Stunden aufgenommen.

Durch Destillation erhält man 450 g Produkt DT-BED (87 % d.Th.).

Beispiel 3

Eine Lösung von t-Butylamin in Octan wird gemäß Beispiel 1 mit wäßrigem Glyoxal umgesetzt. Die Ölphase wird abgetrennt und entwässert. Als Hydrierkontakt dient Raney-Nickel in Octan. Zu seiner Herstellung wird Wasser von Raney-Nickel abgesaugt, Reste mit Methanol ausgewaschen und der Kontakt in Benzin suspendiert. Die Hydrierung geschieht bei 60 bis 80°C und 60 bar Wasserstoff. Die Ausbeute nach Reindestillation ist 85 % d.Th.

## Patentansprüche

1. Verfahren zur Herstellung von Di-tert.-butylethylendiamin durch Umsetzung von tert.-Butylamin mit Glyoxal unter Bildung von Di-tert.-butyl-glyoxaldiimin und Wasser und anschließende Hydrierung des Diimins, dadurch gekennzeichnet, daß man
   (a) die Umsetzung des tert.-Butylamins mit dem Glyoxal in Gegenwart einer Kohlenwasserstoffphase durchführt,
   (b) den Kohlenwasserstoff mit dem Umsetzungsprodukt von Wasser abtrennt, und
   (c) das Umsetzungsprodukt in der Kohlenwasserstoffphase katalytisch hydriert.

2. Verfahren nach Anspruch 1, dadurch gekennzeichnet, daß man das tert.-Butylamin in Kohlenwasserstoff löst und das Glyoxal in wäßriger Lösung einsetzt.

3. Verfahren nach einem der Ansprüche 1 oder 2, dadurch gekennzeichnet, daß man als Kohlenwasserstoffe solche einsetzt, die unter den Bedingungen der Hydrierung des Diimins nicht hydrierbar sind.

4. Verfahren nach einem der Ansprüche 1 bis 3, dadurch gekennzeichnet, daß man Kohlenwasserstoffe mit einem Siedepunkt von 45° bis 160°C einsetzt.

5. Verfahren nach einem der Ansprüche 1 bis 3, dadurch gekennzeichnet, daß man Kohlenwasserstoffe mit einem Siedepunkt zwischen 60 und 130°C einsetzt.

6. Verfahren nach einem der Ansprüche 1 bis 5, dadurch gekennzeichnet, daß man vor der katalytischen Hydrierung des Umsetzungsprodukts gegebenenfalls noch in der Kohlenwasserstoffphase enthaltenes Wasser entfernt.

7. Verfahren nach einem der Ansprüche 1 bis 6, dadurch gekennzeichnet, daß man bei diskontinuierlicher Durchführung des Verfahrens die Hydrierung in Gegenwart eines Suspensionskatalysators durchführt.

8. Verfahren nach einem der Ansprüche 1 bis 6, dadurch gekennzeichnet, daß man bei kontinuierlicher Durchführung des Verfahrens in Gegenwart eines Trägerkatalysators arbeitet.

9. Verfahren nach einem der Ansprüche 1 bis 8, dadurch gekennzeichnet, daß man die Umsetzung des tert.-Butylamins mit Glyoxal bei Raumtemperatur bis 150°C durchführt.

10. Verfahren nach einem der Ansprüche 1 bis 9, dadurch gekennzeichnet, daß man die Hydrierung des Diimins bei Temperaturen zwischen 50 und 150°C, insbesondere 60 bis 80°C, durchführt.

11. Verfahren nach einem der Ansprüche 1 bis 10, dadurch gekennzeichnet, daß man bei Drücken zwischen 10 und 60bar arbeitet.

## Claims

1. A process for preparing di-tert.-butylethylenediamine by reacting tert.-butylamine with glyoxal to form di-tert.-butylglyoxaldiimine and water and subsequently hydrogenating the diimine, which comprises
   (a) carrying out the reaction of tert.-butylamine with glyoxal in the presence of a hydrocarbon phase,

(b) separating the hydrocarbon with the reaction product from water, and

(c) catalytically hydrogenating the reaction product in the hydrocarbon phase.

2. A process as claimed in claim 1, wherein the tert.-butylamine is dissolved in hydrocarbon and the glyoxal is used in the form of an aqueous solution.

3. A process as claimed in either of claims 1 and 2, wherein the hydrocarbon used is of the type which does not undergo hydrogenation under the conditions of hydrogenating the diimine.

4. A process as claimed in any one of claims 1 to 3, wherein the hydrocarbon used has a boiling point of from 45° to 160°C.

5. A process as claimed in any one of claims 1 to 3, wherein the hydrocarbon used has a boiling point of from 60° to 130°C.

6. A process as claimed in any one of claims 1 to 5, wherein any water still present in the hydrocarbon phase is removed before the catalytic hydrogenation of the reaction product.

7. A process as claimed in any one of claims 1 to 6, wherein, if the process is carried out batchwise, the hydrogenation is carried out in the presence of a suspension catalyst.

8. A process as claimed in any one of claims 1 to 6, wherein, if the process is carried out continuously, a supported catalyst is employed.

9. A process as claimed in any one of claims 1 to 8, wherein the reaction of the tert.-butylamine with glyoxal is carried out at from room temperature to 150°C.

10. A process as claimed in any one of claims 1 to 9, wherein the hydrogenation of the diimine is carried out at 50 to 150°C, in particular at 60 to 80°C.

11. A process as claimed in any one of claims 1 to 10, wherein a pressure of from 10 to 60 bar is employed.

## Revendications

1. Procédé de préparation de di-tert.-butyléthylènediamine par réaction de tert.-butylamine et de glyoxal avec formation de di-tert.-butyl-glyoxaldiimine et d'eau, puis par hydrogénation de la diimine, caractérisé en ce que

a) on conduit la réaction de la tert.-butylamine avec le glyoxal en présence d'une phase hydrocarbonée,

(b) on sépare de l'eau l'hydrocarbure avec le produit de la réaction et

(c) on soumet le produit de la réaction à une hydrogénation catalytique dans la phase hydrocarbonée.

2. Procédé selon la revendication 1, caractérisé en ce qu'on dissout la tert.-butylamine dans l'hydrocarbure et on met en réaction le glyoxal en solution aqueuse.

3. Procédé selon la revendication 1 ou 2, caractérisé en ce qu'on utilise, comme hydrocarbures, ceux qui ne sont pas hydrogénables dans les conditions de l'hydrogénation de la diimine.

4. Procédé selon l'une quelconque des revendications 1 à 3, caractérisé en ce qu'on utilise des hydrocarbures ayant un point d'ébullition de 45° à 160°C.

5. Procédé selon l'une quelconque des revendications 1 à 3, caractérisé en ce qu'on utilise des hydrocarbures ayant un point d'ébullition entre 60 et 130°C.

6. Procédé selon l'une quelconque des revendications 1 à 5, caractérisé en ce qu'avant l'hydrogénation catalytique du produit de la réaction, on élimine l'eau encore contenue éventuellement dans la phase hydrocarbonée.

7. Procédé selon l'une quelconque des revendications 1 à 6, caractérisé en ce qu'en cas d'exécution discontinue du procédé, on conduit l'hydrogénation en présence d'un catalyseur en suspension.

8. Procédé selon l'une quelconque des revendications 1 à 6, caractérisé en ce qu'en cas d'exécution continue du procédé, on opère en présence d'un catalyseur fixé sur support.

9. Procédé selon l'une quelconque des revendications 1 à 8, caractérisé en ce qu'on conduit la réaction de la tert.-butylamine avec le glyoxal à une température se situant entre la température ambiante et 150°C.

10. Procédé selon l'une quelconque des revendications 1 à 9, caractérisé en ce qu'on conduit l'hydrogénation de la diimine à des températures comprises entre 50 et 150°C, en particulier entre 60 et 80°C.

11. Procédé selon l'une quelconque des revendications 1 à 10, caractérisé en ce qu'on opère sous des pressions comprises entre 10 et 60 bars.